# EUROPEAN PATENT APPLICATION

(11) **EP 4 756 826 A1**
(43) Date of publication of application: **10.06.2026**
(21) Application number: 25221296.4
(22) Date of filing: 05.12.2025
(51) Int. Cl.: G16H 20/40, A61B 34/10, A61B 34/30, G16H 40/63, A61B 17/04

(54) **ADAPTIVE RETRACTION FORCE CONTROL**

(30) Priority: 06.12.2024 US 202418971888
(71) Applicant: Cilag GmbH International, 6300 Zug (CH)
(72) Inventor: SHELTON IV, Frederick E., Cincinnati, 45242 (US); ADAMS, Shane R., Cincinnati, 45242 (US); COWPERTHWAIT, Matthew David, Cincinnati, 45242 (US); HARRIS, Jason L., Cincinnati, 45242 (US)
(74) Representative: Carpmaels & Ransford LLP

(57) **Abstract**

A surgical system may be associated with cutting and retraction of tissues. The system may include a processor that is configured to work with a surgical cutting instrument and a retraction instrument. The processor may identify a current surgical task, determine the proximity of nearby structures, and calculate a retraction force direction and an intensity. Based on retraction force direction and intensity, the system may generate a control signal that directs the retraction instrument to apply the calculated force, which may allow for the first tissue to be retracted and the second tissue to be cut by the surgical cutting instrument.

## Description

### CROSS-REFERENCE TO RELATED APPLICATIONS

The contents of each of the following, filed contemporaneously, are incorporated by reference herein:
- Attorney Docket No. END9638USNP1, entitled PROGRESSIVE ADVANCEMENT OF AUTOMATED LEVEL BASED ON LEARNED COMPLIMENTARY ASSISTANCE
- Attorney Docket No. END9638USNP2, entitled ADJUSTING AUTOMATED COOPERATIVE OPERATIONS BASED ON SITUATIONALLY DERIVED CONSTRAINTS,
- Attorney Docket No. END9638USNP3, entitled ASSISTANCE ADVANCEMENT MULTI-SYSTEM INTERACTION,
- Attorney Docket No. END9638USNP4, entitled MONITORING AND IDENTIFYING SURGEON CONTROL AND SUGGESTING A TASK THAT MAY BE DONE AUTONOMOUSLY,
- Attorney Docket No. END9638USNP5, entitled CONTROL OF INFORMATION FLOW, PRIORITIZATION AND MANIFESTATION OF DATA ASSOCIATED WITH AN ACTIVE HCP INTERACTION SPACE,
- Attorney Docket No. END9638USNP7, entitled ADJUSTMENT OR DISPLAY OF OPTIONS OF POSITIONAL OR ORIENTATION IMPLICATIONS ON SURGICAL TOOL USAGE, and
- Attorney Docket No. END9638USNP8, entitled ADJUSTMENT OF PHYSIOLOGIC FUNCTION SUPPLEMENTATION CONTROL.

The contents of each of the following are incorporated by reference herein:
- U.S. Patent Application No. 18/810,323 entitled METHOD FOR MULTI-SYSTEM INTERACTION, filed on August 20, 2024;
- U.S. Patent Application No. 18/960,006 entitled METHOD FOR SMART SURGICAL SYSTEMS filed on November 26, 2024; and
- U.S. Patent Application No. 18/954,186 entitled METHOD FOR MULTI-SYSTEM INTERACTION, filed on November 20, 2024.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

A surgical system may be associated with cutting and retraction of tissues. The system may include a processor that is configured to work with a surgical cutting instrument and a retraction instrument. The processor may identify a current surgical task, determine the proximity of nearby structures, and calculate a retraction force direction and an intensity. Based on retraction force direction and intensity, the system may generate a control signal that directs the retraction instrument to apply the calculated force, which may allow for the first tissue to be retracted and the second tissue to be cut by the surgical cutting instrument.

The surgical system may calculate the movement direction and speed of the cutting instrument and predict how the tissue may respond to the movements. By considering the proximity of nearby structures, the system may adjust the retraction force to avoid causing injury. The system may cause the cutting instrument to modify the movement of the cutting instrument based on the calculated retraction force or direction indicating a likelihood (e.g., a high likelihood) of harm to surrounding tissues.

The system may determine tension ranges and retraction forces for the tissues being operated on. The system may prevent tissue damage based on the tension ranges and retraction forces, such as preventing tearing. The system may also incorporate preselected force intensity limits, and the preselected force intensity limits may enable the retraction instrument to not exceed a force threshold.

The system may use imaging to identify tissue planes and critical structures. The identified tissue planes and critical structures may allow the system to adjust the forces applied during retraction and dissection to minimize the risk of injury, for example, when working with scarred or remodeled tissue. The system may modify control signals based on the identified tissue planes and critical structures to enable the surgical instruments to move through the target area.

### BRIEF DESCRIPTION OF THE DRAWINGS

FIG. 1 is a block diagram of a computer-implemented surgical system.
FIG. 2 shows an example surgical system in a surgical operating room.
FIG. 3 illustrates an example surgical hub paired with various systems.
FIG. 4 shows an example situationally aware surgical system, and FIG. 5 shows an example surgical instrument.
FIG. 6A illustrates an example stage of the surgical process where a retraction instrument pulls at a first target tissue away from a surgical site to provide visibility.
FIG. 6B illustrates example stage of the surgical process where connective tissue is partially cut by a laparoscopic bipolar dissecting device and a retraction instrument that applies force to retract stomach tissue.
FIG. 6C illustrates an ongoing retraction of the stomach as more of the connective tissue is dissected.
FIG. 7 illustrates an example flow diagram of a surgical system process.
FIG. 8 illustrates an example machine learning-based diagram associated with the surgical system.

### DETAILED DESCRIPTION

A more detailed understanding may be had from the following description, given by way of example in conjunction with the accompanying drawings.

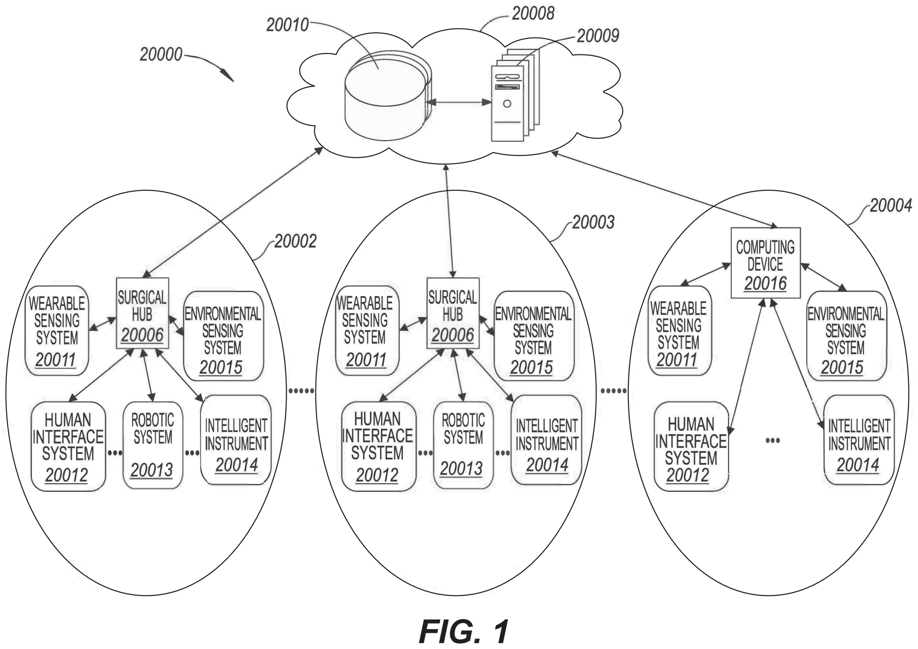

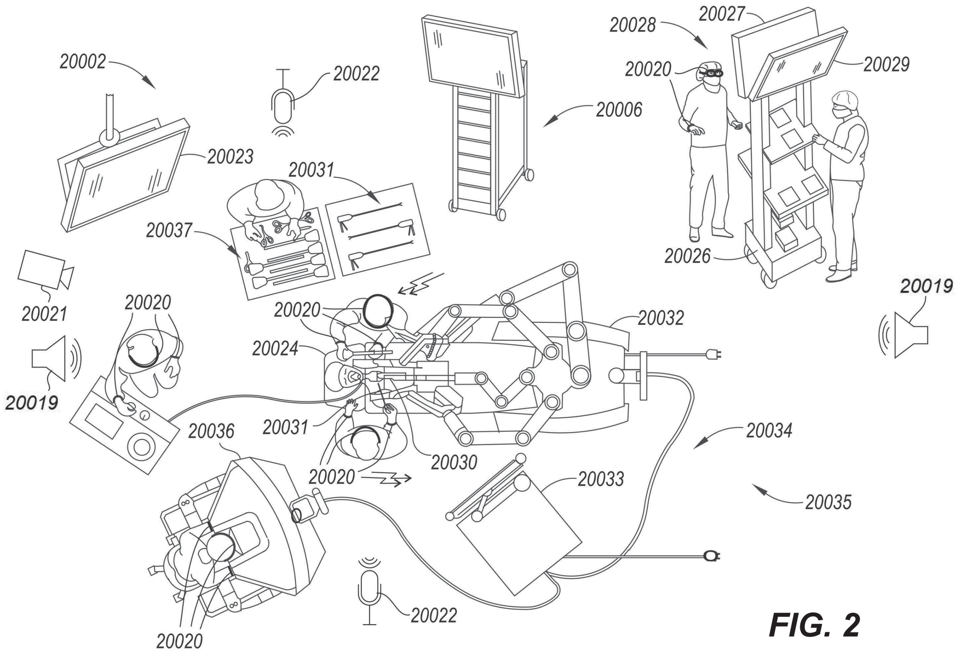

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more health care professional (HCP) sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include, tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, which is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

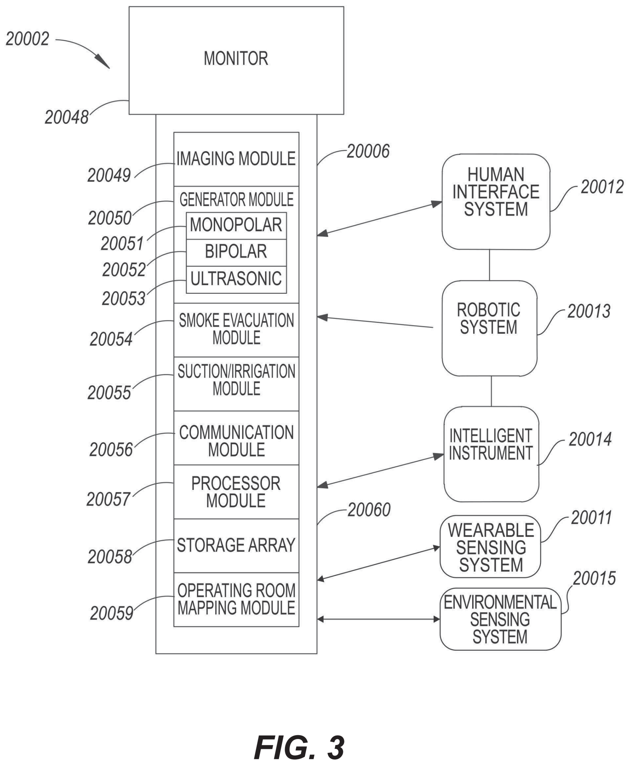

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

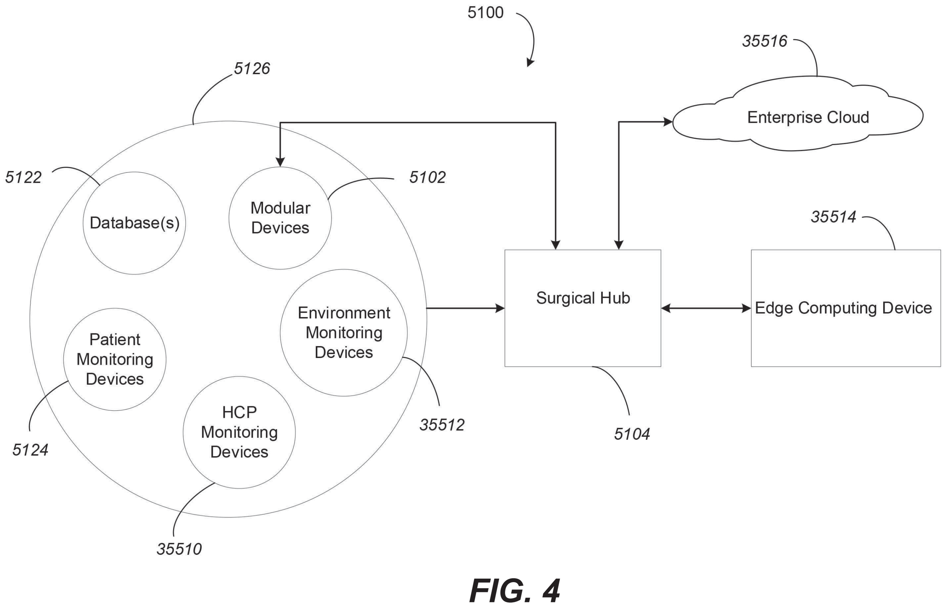

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an RF electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedure-by-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

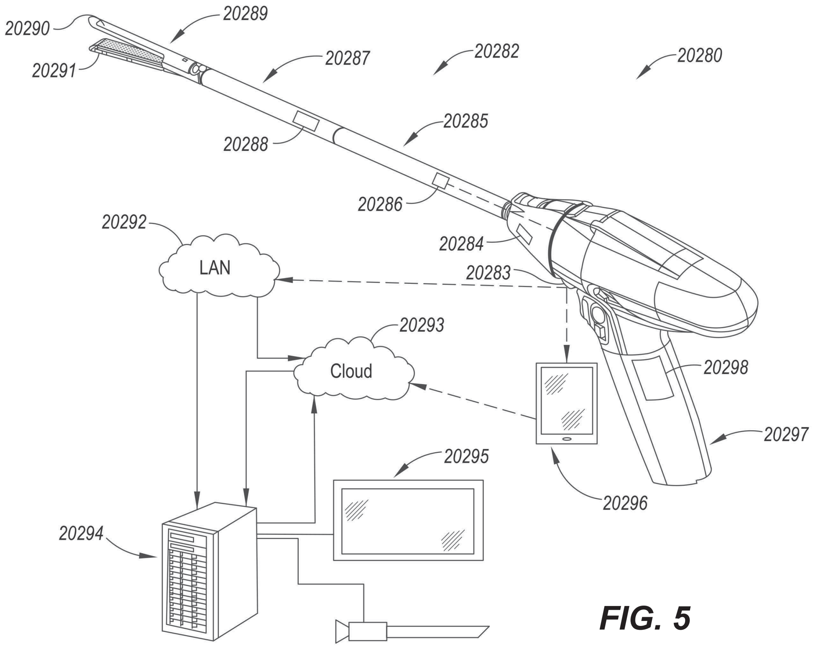

FIG. 5A illustrates an example surgical system 20280 that may include a surgical instrument 20282. The surgical instrument 20282 can be in communication with a console 20294 and/or a portable device 20296 through a local area network 20292 and/or a cloud network 20293 via a wired and/or wireless connection. The console 20294 and the portable device 20296 may be any suitable computing device. Surgical instrument 20282 may include a handle 20297, an adapter 20285, and a loading unit 20287. The adapter 20285 releasably couples to the handle 20297 and the loading unit 20287 releasably couples to the adapter 20285 such that the adapter 20285 transmits a force from a drive shaft to the loading unit 20287. The adapter 20285 or the loading unit 20287 may include a force gauge (not explicitly shown) disposed therein to measure a force exerted on the loading unit 20287. The loading unit 20287 may include an end effector 20289 having a first jaw 20291 and a second jaw 20290. The loading unit 20287 may be an in-situ loaded or multi-firing loading unit (MFLU) that allows a clinician to fire a plurality of fasteners multiple times without requiring the loading unit 20287 to be removed from a surgical site to reload the loading unit 20287.

The first and second jaws 20291, 20290 may be configured to clamp tissue therebetween, fire fasteners through the clamped tissue, and sever the clamped tissue. The first jaw 20291 may be configured to fire at least one fastener a plurality of times or may be configured to include a replaceable multi-fire fastener cartridge including a plurality of fasteners (e.g., staples, clips, etc.) that may be fired more than one time prior to being replaced. The second jaw 20290 may include an anvil that deforms or otherwise secures the fasteners, as the fasteners are ejected from the multi-fire fastener cartridge.

The handle 20297 may include a motor that is coupled to the drive shaft to affect rotation of the drive shaft. The handle 20297 may include a control interface to selectively activate the motor. The control interface may include buttons, switches, levers, sliders, touchscreens, and any other suitable input mechanisms or user interfaces, which can be engaged by a clinician to activate the motor.

The control interface of the handle 20297 may be in communication with a controller 20298 of the handle 20297 to selectively activate the motor to affect rotation of the drive shafts. The controller 20298 may be disposed within the handle 20297 and may be configured to receive input from the control interface and adapter data from the adapter 20285 or loading unit data from the loading unit 20287. The controller 20298 may analyze the input from the control interface and the data received from the adapter 20285 and/or loading unit 20287 to selectively activate the motor. The handle 20297 may also include a display that is viewable by a clinician during use of the handle 20297. The display may be configured to display portions of the adapter or loading unit data before, during, or after firing of the instrument 20282.

The adapter 20285 may include an adapter identification device 20284 disposed therein and the loading unit 20287 may include a loading unit identification device 20288 disposed therein. The adapter identification device 20284 may be in communication with the controller 20298, and the loading unit identification device 20288 may be in communication with the controller 20298. It will be appreciated that the loading unit identification device 20288 may be in communication with the adapter identification device 20284, which relays or passes communication from the loading unit identification device 20288 to the controller 20298.

The adapter 20285 may also include a plurality of sensors 20286 (one shown) disposed thereabout to detect various conditions of the adapter 20285 or of the environment (e.g., if the adapter 20285 is connected to a loading unit, if the adapter 20285 is connected to a handle, if the drive shafts are rotating, the torque of the drive shafts, the strain of the drive shafts, the temperature within the adapter 20285, a number of firings of the adapter 20285, a peak force of the adapter 20285 during firing, a total amount of force applied to the adapter 20285, a peak retraction force of the adapter 20285, a number of pauses of the adapter 20285 during firing, etc.). The plurality of sensors 20286 may provide an input to the adapter identification device 20284 in the form of data signals. The data signals of the plurality of sensors 20286 may be stored within or be used to update the adapter data stored within the adapter identification device 20284. The data signals of the plurality of sensors 20286 may be analog or digital. The plurality of sensors 20286 may include a force gauge to measure a force exerted on the loading unit 20287 during firing.

The handle 20297 and the adapter 20285 can be configured to interconnect the adapter identification device 20284 and the loading unit identification device 20288 with the controller 20298 via an electrical interface. The electrical interface may be a direct electrical interface (i.e., include electrical contacts that engage one another to transmit energy and signals therebetween). Additionally, or alternatively, the electrical interface may be a non-contact electrical interface to wirelessly transmit energy and signals therebetween (e.g., inductively transfer). It is also contemplated that the adapter identification device 20284 and the controller 20298 may be in wireless communication with one another via a wireless connection separate from the electrical interface.

The handle 20297 may include a transceiver 20283 that is configured to transmit instrument data from the controller 20298 to other components of the system 20280 (e.g., the LAN 20292, the cloud 20293, the console 20294, or the portable device 20296). The controller 20298 may also transmit instrument data and/or measurement data associated with one or more sensors 20286 to a surgical hub. The transceiver 20283 may receive data (e.g., cartridge data, loading unit data, adapter data, or other notifications) from the surgical hub 20270. The transceiver 20283 may receive data (e.g., cartridge data, loading unit data, or adapter data) from the other components of the system 20280. For example, the controller 20298 may transmit instrument data including a serial number of an attached adapter (e.g., adapter 20285) attached to the handle 20297, a serial number of a loading unit (e.g., loading unit 20287) attached to the adapter 20285, and a serial number of a multi-fire fastener cartridge loaded into the loading unit to the console 20294. Thereafter, the console 20294 may transmit data (e.g., cartridge data, loading unit data, or adapter data) associated with the attached cartridge, loading unit, and adapter, respectively, back to the controller 20298. The controller 20298 can display messages on the local instrument display or transmit the message, via transceiver 20283, to the console 20294 or the portable device 20296 to display the message on the display 20295 or portable device screen, respectively.

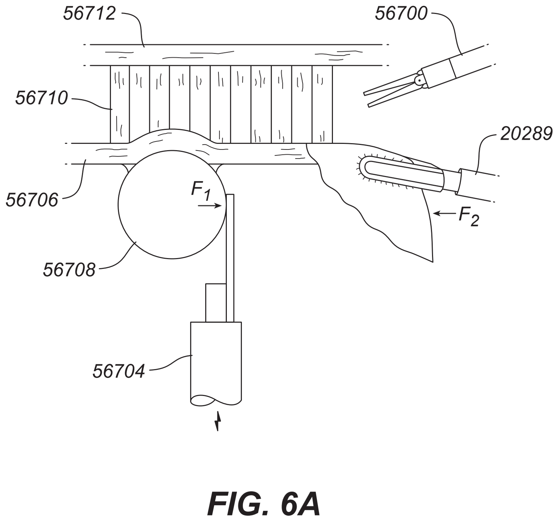

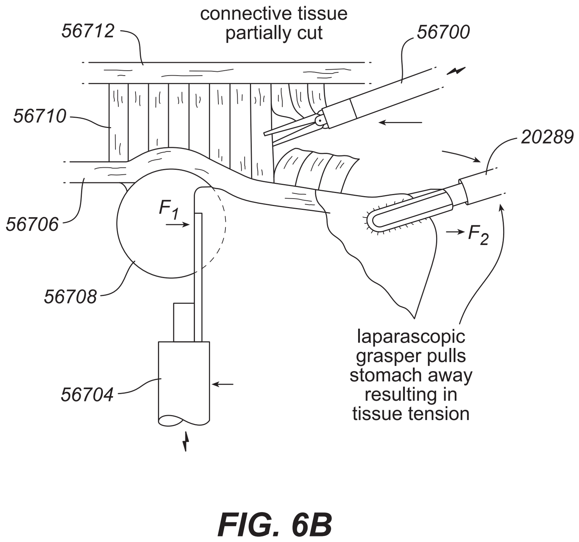

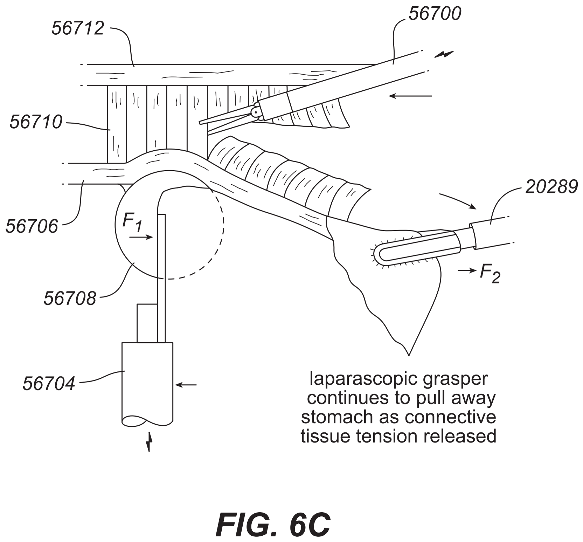

FIGS. 6A, 6B, and 6C illustrate a surgical system configured for the manipulation of tissues during a laparoscopic procedure. Features described herein may be associated with an interaction between a laparoscopic bipolar dissecting device 56700 and a laparoscopic grasper 20289 working together to perform tissue retraction and cutting near the stomach and diaphragm during a surgical procedure.

The surgical system may function as one or more of a central hub or as an example system controlling the retraction instrument. For example, as a hub, the system may coordinate multiple instruments, such as the cutting and retraction tools. The system may focus on managing retraction force, direction, and tissue displacement to protect surrounding structures. The system may adjust based on real-time feedback.

In FIG. 6A, the surgical system may include a step in the surgical process, for example, the laparoscopic grasper 20289 pulling the stomach tissue while the laparoscopic bipolar dissecting device 56700 cooperatively releases connective tissue around the stomach 56706 and diaphragm 56712. The laparoscopic instruments may act together in proximity to neighboring structures, for example, the stomach wall 56706 and connective tissue 56710, such that injury to the surrounding anatomy is avoided. For example, the system may identify the current task, determine the proximity of neighboring structures, and calculate the retraction force associated cutting (e.g., surrounding tissues/connective tissue) without causing damage.

The current task may include procedures such as mobilization, skeletonization, and dissection. Mobilization may include the controlled movement and positioning of tissues to provide access to the surgical site. Skeletonization may include separation of tissues to expose important anatomical structures, such as blood vessels or organs, while minimizing damage to surrounding areas. Dissection may include cutting and removal of targeted tissues. Throughout one or more of the procedures, the system may monitor tissue tension and proximity to neighboring structures, for example, dynamically adjusting retraction forces and cutting actions for precision and reducing the risk of injury.

FIG. 6B illustrates a step in the surgical process, for example, where the connective tissue 56710 is partially cut, and the laparoscopic grasper 20289 continues to exert a retraction force F2. As the force pulls the stomach tissue 56706 away, the system may calculate the movement of the grasper, determine the proximity of the neighboring structures, and generate control signals to adjust the retraction force in real-time. The forces may be balanced, such that tissues are moved to maintain tension and allowed for controlled cutting by the laparoscopic bipolar dissecting device 56700.

FIG. 6C illustrates a step in the surgical process, where the laparoscopic grasper 20289 maintains the retraction force F2 on the stomach 56706 as more of the connective tissue 56710 is released. The surgical system may monitor the displacement of target tissue and determine whether the applied force may cause potential injury to adjacent structures. The system may correspond control signals to modify the retraction or cutting movement based on the likelihood of injury.

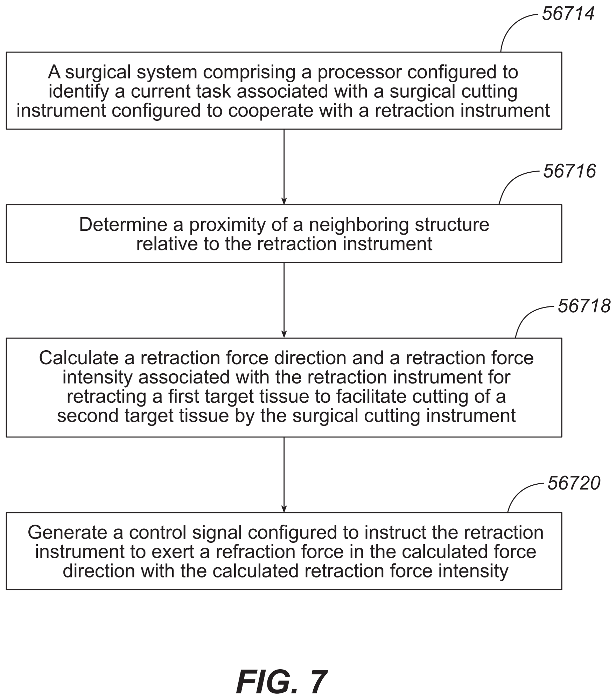

Fig. 7 illustrates an example diagram associated with operation of a surgical system. At 56714, the system may identify a current task associated with a surgical cutting instrument configured to cooperate with a retraction instrument. For example, the system may analyze the procedure and take into consideration goals of the operation and the interaction between the cutting and retraction instruments.

In identifying the current task associated with the surgical cutting instrument, the system may use the following information. The system may analyze the surgical context information, which may include the type of procedure being performed, the target tissues involved, and the surrounding anatomical structures. The system may determine the task based on whether the surgical cutting instrument is energized or not, as the energization status of the instrument may indicate whether the instrument is ready to perform cutting, cauterizing, or other actions. The system may consider the motion of the surgical cutting instrument, such as its direction, speed, or position relative to the retraction instrument, to identify the current task and coordinate the actions of the surgical cutting instrument with the retraction instrument.

For example, during laparoscopic procedures, as illustrated in FIGS. 6A-C, the surgical cutting instrument 56700 may be positioned to cut tissue around the stomach 56706 and diaphragm 56712, and the retraction instrument 20289 may pull away the stomach tissue. The system may analyze the target tissue and surgical site and determine the role of an instrument in the context of the surgical procedure. The system may determine a proximity of neighboring structures and a task, such as cutting connective tissue while mitigating damage to nearby structures (e.g., organs/unassociated tissue).

In examples, the surgical cutting instrument 56700 may include a smart energy device. An example energy device may be configured to perform cutting, dissection, or coagulation tasks. An example energy device may use electrosurgical techniques for tissue cutting. An example energy device may use ultrasonic technology for tissue dissection. The example energy devices may be used for the purposes of and in association with reducing bleeding or improving tissue healing.

Features described herein may be associated with imaging or identifying a smart device for tissue separation and energy adjustment. The imaging or identifying smart device may show the path of a previous tissue plane. Based on data from the identification system, the device may adjust tissue separation forces, motions, or the magnitude of energy used in an energy supplement dissection. The adjustments may be based on on how close the device is to critical structures or how far the device is from the expected path. The system may use such data to adapt force application or generator power levels based on the proximity of the previous tissue plane.

A force or generator power level may be adapted based on a proximity of and occluded tissue plane. The system may adapt the force or generator power level based on how tissue scarring affects tissue properties. For example, tougher, scarred tissue may be associated with higher power levels to cut through. As the power level increases, the risk of making overly deep cuts may increase, which may puncture underlying critical structures. The system may adjust the response based on tissue scarring and apply more aggressive energy power the closer the energized device is to the original tissue plane and reduce energy levels when operating near critical structures.

Energy applied may be limited based on device pressure for initial dissection points. The energy applied by the device may be limited based on the pressure exerted during the procedure. The energized device may create initial dissection points, which may lower the respective force associated with mechanical instruments during tissue dissection. For example, during a colorectal procedure, a surgeon may extend the circular stapler trocar to tent the tissue before creating an anastomosis. The surgeon may manually use a monopolar pencil to assist the trocar, limiting forces associated with the surgical procedure. A cooperative system may utilize monopolar devices or (e.g., other) energy tools to establish dissection points and reduce the mechanical force associated with further dissection.

Abrasive dissection (e.g., performed with a peanut dissector) may be based on the abrasive properties of the instrument's head and the forces applied to separate tissue mechanically. The force applied may be proportionate to the depth of dissection per pass. As direction of the pass becomes more aggressive, the depth of dissection may become greater, and the risk of damaging underlying critical structures may increase. As the dissection nears the original (e.g., lost tissue path), the surgeon may be more aggressive without increasing the risk of unintended damage. Critical structures may not cross tissue planes, and if they may, they may do so in predictable locations.

In examples where blunt dissection is performed, such as with a peanut or an abrasive dissecting system, the force application may depend on the amount of lateral motion relative to the force applied. The system may utilize energy electrical parameters, such as tissue impedance, as a proxy for mechanical force. The proxy parameter may limit mechanical force application, such that excessive pressure is not applied.

Electrical Impedance may be used as a proxy for tissue compression. For example, in examples associated with a bipolar device, the system may use electrical impedance to inform the tissue characteristics and determine the mechanical forces associated with stapler firing, allowing the surgeon to adjust the applied forces based on how easily the tissue separates once the jaws are inserted. The forces applied, the rate of separation, and the ease with which the jaws open and separate tissue may be associated with determining how to configure the dissection process, such as when tissue characteristics vary due to scarring or other abnormalities.

The retraction instrument 20289 may include retraction devices and, for example, a retraction grasper. The retraction grasper may be configured to manipulate and retract tissue to expose the target area for the surgical cutting instrument. The retraction devices may be used to adjust tissue positioning in real time, based on the control signals generated by the system. The instruments 56700 and 20289 may work in tandem with the surgical cutting device during stages of the surgical procedure.

At 56716, the system may determine a proximity of a neighboring structure relative to the retraction instrument. The proximity of the structures may be monitored by the system to enable the calculated forces applied during retraction and cutting to not lead to unintended injury. For example, in FIG. 6A, the connective tissue 56710 and the stomach wall 56706 may be close to the surgical site where the laparoscopic instruments are operating. The system may determine a distance between the neighboring tissues and the retraction instrument to avoid overstretching or tearing while retracting the stomach tissue. The real-time assessment may enable the retraction forces applied to a target tissue (e.g., the stomach) to be controlled. The system may update its calculations as the cutting instrument proceeds with the surgical task.

At 56718, the system may calculate a retraction force direction and a retraction force intensity associated with the retraction instrument for retracting a first target tissue to facilitate cutting of a second target tissue by the surgical cutting instrument (e.g., based on the identified task and the proximity of neighboring structures).

In examples, the system may be configured to manipulate a first target tissue and a second target tissue during a surgical procedure. The first target tissue may include the organ that is being mobilized, such as the stomach, liver, or another anatomical structure. The second target tissue may include the tissues surrounding the organ that are intended to be cut or dissected during the mobilization process. For example, during the mobilization of the stomach, the second target tissue may include connective tissues or ligaments that are to be severed in order to safely reposition the organ. The system may be designed to apply precise retraction forces on the first target tissue (the organ) to expose the second target tissue for cutting, such that the organ is manipulated (e.g., effectively manipulated) while the surrounding tissues are dissected.

For example, in FIG. 6B, the laparoscopic grasper 20289 may exert a retraction force F2 on the stomach tissue 56706 while the connective tissue 56710 is partially cut. The system may calculate the direction in which the stomach tissue should be pulled, as well as the appropriate intensity of the force associated with keeping the tissue taut without causing excessive tension that could lead to injury. The calculation may take into account the proximity of structures and the movement of the cutting instrument and the rate of tissue displacement. The system may provide the force for the cutting instrument to proceed with the operation while avoiding excessive pressure on surrounding tissue.

At 56720, the system may generate a control signal to instruct the retraction instrument to exert a retraction force in the calculated retraction force direction with the calculated retraction force intensity. In FIG. 6C, for example, the laparoscopic grasper 20289 may maintain a retraction force F2 on the stomach tissue 56706 as more connective tissue 56710 is released. The control signal generated may guide the retraction instrument to continue pulling the stomach tissue with the calculated force, such that the tissue remains in the proper position for the cutting instrument to operate safely and efficiently.

The system may adjust the control signal in response to a change in the surgical environment, such as tissue displacement or proximity to neighboring structures. For example, if the system detects a shift in the position of the stomach or nearby tissues, the system may recalibrate the retraction force in real-time to maintain effective retraction.

In examples, the surgical system may determine a movement direction and a movement speed associated with the surgical cutting instrument as the surgical cutting instrument cuts the second target tissue at a first time instance. The system may calculate a target displacement of the first target tissue at a second time instance based on the movement direction and the movement speed associated with the surgical cutting instrument. The system may calculate, based on the target displacement of the first target tissue and the proximity of the neighboring structure relative to the retraction instrument, a probability that retracting the first target tissue to the target displacement would injure the neighboring structure. The system may generate a second control signal to modify the retraction force based on the calculated probability.

In examples, the surgical system may generate a third control signal to instruct the surgical cutting instrument to modify at least one of the movement direction or the movement speed.

In examples, the surgical system may generate an indication configured to indicate a likelihood of injury to the neighboring structure caused by the retraction instrument should the surgical cutting instrument maintain the current movement direction and the current movement speed.

In examples, the surgical system may determine, based on the identified current task associated with the surgical cutting instrument, a target tissue tension range associated with the second target tissue.

Referring back to FIGS. 6A-C, the second target tissue is illustrated as the connective tissue 56710 that the dissecting device 56700 is configured to dissect. Tissue retraction may be associated with clinical purposes. An example clinical purpose, as seen in FIG. 6A, the retraction instrument 20289 may pull the stomach tissue 56706 away from the surgical site, moving the organ out of the visualization path to provide the surgeon with a clear view of the surgical area. Moving the organ out of the visualization path may facilitate visibility for the surgeon to perform dissection of the connective tissue. In examples where retraction is to be progressive, or where additional retraction is used as the organ is progressively freed from its attachments, the system may apply a minimal retraction force or execute a step-wise displacement to enable the organ to remain securely retracted as its fixations are relieved.

Tissue retraction may create a stable grounding force for the surgical instruments, such as the laparoscopic bipolar dissecting device 56700, to work against. As illustrated in FIG. 6B, as the retraction instrument applies force F2 to the stomach, tension is generated in the surrounding tissues, providing a counterforce for the dissecting instrument to separate and cut the connective tissue 56710. The grounding force allows the retraction instrument to create a tension to support the dissection and support precision in separating the second target tissue (e.g., connective tissue) from the first target tissue (e.g., organ), as seen in FIG. 6C.

The system may determine a maximum retraction force intensity based on the determined target tension range associated with the second tissue to avoid tearing of the second target tissue, wherein the retraction force intensity associated with the retraction instrument is calculated based on the determined maximum retraction force intensity.

In examples, the surgical system may determine, based on the identified current task associated with the surgical cutting instrument, a target displacement associated with the first target tissue such that the second target tissue is visible for cutting by the surgical cutting instrument. The system may determine a minimum retraction force intensity based on the determined target displacement associated with the first target tissue, wherein the retraction force intensity associated with the retraction instrument is calculated based on the determined minimum retraction force intensity.

In examples, the surgical system may perform clinical tasks related to tissue retraction. Organ retraction may be associated with moving an organ or part of an organ out of the visualization path, providing the surgeon with a clear view of the surgical site. This retraction may be progressive and associated with additional retraction force as the organ is gradually freed from its attachments. In examples, the system may calculate a minimum retraction force or perform step-wise displacements to enable the organ to be retracted in a controlled manner, minimizing the risk of damage as the organ's fixations are relieved. An example clinical task may include creating a grounding force for the retraction instrument to work against, allowing the instrument to separate the second target tissue, such as connective tissue, from the first target tissue (e.g., the organ). By calculating a target displacement of the first target tissue, the system may enable the second target tissue to be properly positioned for cutting. The system may determine the minimum retraction force intensity associated with achieving the displacement.

In examples, the surgical system may obtain a preselected force intensity limit associated with the retraction instrument, wherein the retraction force intensity associated with the retraction instrument is calculated based further based on the preselected force intensity limit.

In examples, the surgical system may adapt the instrument loading based on both the system's awareness of the identified task, user control actions, and a preselected force intensity limit. The system may obtain the preselected force intensity limit associated with the retraction instrument and calculate the retraction force intensity accordingly. By monitoring the surgical task, such as cutting or dissecting the second target tissue, and recognizing the user's control inputs, the system may adjust the applied force, so that the applied force remains within the defined limits. The dynamic adaptation of retraction force may cause the instruments to minimize the risk of tissue damage while maintaining the force to manipulate the first target tissue. Additionally, the system may utilize the preselected force intensity limit to create a controlled interaction between the instruments.

Features described herein may be associated with a controlled limitation of applied forces to the tissue, to achieve the desired tissue interaction. The system described herein may provide a controlled limitation of applied forces to the tissue during surgical procedures to achieve the desired tissue interaction. The control may be based on an adaptation of the instrument loading, which may take into consideration factors, including the awareness of the current task, user control actions, and a preselected force limit or coupled displacement. The system may adjust the force limits. The system may use situational awareness of the operation and consider how the user manipulates the instrument relative to surrounding tissues. The system may display the current force applied or the force threshold in relation to predefined input limits and provide feedback to the user to prevent excessive force during surgical procedures. The system may display directional information about the applied forces. Future motions may be directed, and forces may be displayed in a manner that benefits sequential surgical operations.

Features described herein may be associated with tissue retraction and control to provide access to surgical intervention sites. The retraction device may adaptively control both the direction and intensity of the reaction force. The system may maintain the tissue in a stationary position or apply the retraction force to facilitate interaction with other surgical devices.

Clinical jobs may be associated with tissue retraction. Tissue retraction during surgery may fulfill clinical functions. Organ retraction may be associated with moving an organ or part of an organ out of the surgical field, providing the surgeon with a clear view of the operative area. The system may control the retraction for a progressive or step-wise displacement of the organ as attachments of the organ are released. The system may calculate and apply a minimum necessary force for retraction to avoid unnecessary tension or injury. Tissue retraction may provide a grounding force against which the retraction instrument may function, which may promote tissue separation. The system may balance the retraction force between a window of acceptable values, such that the retraction force is enough to move the organ without causing damage, and such that the user is aware of the proximity of adjacent tissue structures.

Features described herein may be associated with progressive visualization and counter-retraction. The system may account for reactive force and displacement during tissue counter-retraction, a process where the force applied by the retraction tool is proportionate to the displacement force applied by a counter-retraction device. The proportional application of force may enable the tissue to be moved in a direction without becoming unintentionally separated. The system may continuously monitor the retraction and counter-retraction forces, such that the counter-retraction forces remain within safe limits and do not interfere with critical adjacent structures. For example, if a neighboring organ or critical tissue is in close proximity, the system may limit the motion of the retraction device to avoid accidental contact or injury.

The system may incorporate situational awareness of the operation of the retraction instrument relative to the surgical cutting instrument. The system may determine a movement direction and movement speed of the surgical cutting instrument as the surgical cutting instrument cuts the second target tissue. Based on the identified task, the system may determine a coupled displacement between the cutting and retraction instruments and adjust the retraction force thresholds. The adaptation of the force threshold or control may be based on an awareness of how the instruments interact during surgery. The system may display the force or its threshold relative to a predefined input limit.

Features described herein may be associated with tissue dissection and/or separation enablement. The system may enable tissue dissection and separation during surgical procedures. Tissue dissection and separation may aid in identifying and navigating tissue planes while minimizing damage to critical structures. The system may allow for the separation of tissues to expose important surgical sites without applying excessive force. Tissue dissection and separation may be associated with complex tissue structures and/or situations where tissue planes are not (e.g., not easily) distinguishable, such as, for example, tissue remodeling or scarring. The system may guide the surgeon in tissue dissection by identifying appropriate separation lines (e.g., while maintaining the structural integrity of the tissues being operated on).

Features described herein may be associated with identification of tissue planes to improve traversing to the desired surgical site while minimizing accidental critical structure damage. The system may be configured to identify tissue planes. By detecting tissue planes and distinguishing the tissue planes from convoluted planes caused by adhesions, scarring, or remodeling, the system may determine the dissection path. The system may provide visual or tactile feedback to assist the surgeon in orienting instruments and maintaining an appropriate trajectory toward the surgical site.

Features described herein may be associated with identification of original tissue planes from convoluted tissue planes. In examples associated with adhesions, disorganized remodeling, or scarring, the system may assist in identifying original tissue planes that may have been obscured by the adhesions, disorganized remodeling, or scarring. The system may use imaging technology to show the surgeon where the natural separation line or tissue plane is located. Once tissue separation begins, the system may provide guidance by indicating where critical structures are located, allowing the surgeon to avoid the critical structures while dissecting. The system may suggest augmentations using energy-based devices, rather than relying on force-driven methods, to assist with tissue separation.

The system may help navigate conditions that complicate tissue separation. For example, when tissue is not separating as healthy tissue may, the system may guide the surgeon through alternative approaches. In examples involving vascularized tissue or an enlarged blood supply, the system may assist by providing information about how to proceed without causing unnecessary damage. In examples involving sensitive structures, such as the liver casing, for example, the system may help maintain the integrity of the casing by identifying how to separate surrounding tissues safely without compromising the structure of the liver itself.

Features described herein may be associated with identification of underlying critical structures. The system may assist surgeons in identifying critical structures located beneath the surface while dissecting along known tissue planes. This may be associated with working along or among adhesions or convoluted tissue planes where the anatomical structures are obscured. Imaging may be used to re-establish the location of the tissue planes being followed during procedures such as skeletonization or tissue mobilization.

The system may be configured for mapping vascularization, which may be associated with determining the safest dissection path. Vascular structures may not cross tissue planes. If vascular structures do cross tissue planes, such as in remodeled tissue areas, the vessels may be smaller compared to organ tissue (e.g., that has not been remodeled). The absence of interconnections or changes (e.g., significant changes) in vessel configuration, size, or trajectory may be used by the system to determine tissue planes when tissue planes are not easily visible. Using multi-spectral imaging, the system may detect tissue planes by identifying dissimilarities in fiber bundles, tissue composition, or reflectivity. The detection of tissue planes (e.g., via identifying dissimilarities in fiber bundles, tissue composition, or reflectivity) may allow the surgeon to visually follow the tissue planes or relocate them after encountering convoluted tissue remodeling.

Dissection may occur in an unintended plane and based on surgeon technical errors. The system may help maintain the relative position to anatomy during laparoscopic and robotic procedures, such as when orientation changes occur throughout the surgery. Maintaining the relative position may be associated with a reduction in inadvertent tissue injury or dissection in the unintended tissue plane. For example, if a surgeon mistakenly dissects in the unintended plane, the system may detect the error and maintain a tracking point on the correct tissue type or plane throughout the procedure. A tag may be dropped, and the system may auto-track the point or tissue type, such that the surgeon remains on the correct surgical path.

Features described herein may be associated with limiting local application and residual Tissue tension. The system may limit local application and residual tissue tension during procedures by monitoring organ or connective tissue that has not been sufficiently mobilized from surrounding fixations or adhesions. When excessive force is applied, tears, perforations, or localized bleeding may occur at fixation or grasping points. The system may adjust force limits to avoid such outcomes by calculating the required tension for manipulation (e.g., safe manipulation).

Features described herein may be associated with communication of tissue tension and instrument applied forces. The system may calculate and communicate the impacts of instrument-applied forces on blood flow and tissue tension. Such forces may be displayed to the user or communicated (e.g., algorithmically) to limit the instrument's operation. The system may provide real-time feedback to the surgeon about tissue tension, forces, and their implications for tissue configuration, blood flow, and healing. Communication between smart systems within the surgical setup may further limit forces to avoid inadvertent damage to tissues.

Features described herein may be associated with the use of motors and proxy measurements for end-effector control. In patent US 9,700,309, four independent motors are utilized to control the end-effector's closure, firing, articulation, and distal head rotation. Building on this architecture, motor current (e.g., force) or the back drive of the unpowered system (e.g., stroke) may be employed to determine the lateral force applied externally to the end-effector or during the active motion of the head. The resulting load, or the load needed to activate articulation or shaft rotation, may serve as a proxy for the external forces (e.g., tension) created or the amount of force remaining after positioning. The system may record the data and transfer it from the smart endocutter to a monitoring system, for example, a visualization hub, which may compare the data with other system inputs.

For visualization, real-time blood flow impacts may be detected using laser doppler flowmetry, intraoperative indocyanine green (ICG) fluorescence angiography, or infrared (IR) thermography. The imaging techniques may operate using wavelengths of light to stimulate or measure active or passive emissions from tissue. The measured blood flow may be compared with the forces and strokes recorded by the device. The data exchange may occur device-to-system, system-to-device, or bi-directionally between devices.

Features described herein may be associated with residual arterial tension due to organ mobilization. In examples, insufficient mobilization of organs (e.g., like the colon), which have connective tissue interconnections and surrounding blood supply, may lead to residual arterial tension. The tension may cause occlusion, collapse, or insufficient blood flow, as the attachment point is pulled back to the anatomy. When residual tension exists in the tissue overlaying the organ and surrounding arteries or capillaries, vessel collapse may occur, restricting blood flow to the organ and leading to ischemia or tissue death. Proper mobilization may be associated with relieving the tensions and enabling adequate blood flow during surgery.

Features described herein may be associated with a residual organ wall or muscular tension inhibiting function. Residual muscular or organ wall tension, caused by stretching from the fixation point, may inhibit internal operations such as peristalsis or lymphatic flow. Peristalsis refers to the involuntary constriction and relaxation of intestinal muscles, creating wave-like movements that propel contents forward. The lymphatic vessels, responsible for collecting and draining interstitial fluid, may function similarly to veins, with thin walls and low pressure. The system may enable adequate drainage to prevent excess fluid accumulation, which may otherwise lead to tissue swelling, compression of blood vessels, and tissue damage.

The larger lymphatic vessels may be equipped with a type of muscle, structurally intermediate between smooth and cardiac muscle, which may contract rhythmically (e.g., like peristalsis) to maintain lymph movement. The contractions may enable continuous movement of lymph, to complement bodily movement and maintain flow while the body is stationary.

Features described herein may be associated with localized tissue tension and staple line management. Localized tissue tension between layers or fiber bundles may cause direct tissue compression, resulting in shear forces, tissue separation, local tears, or compressive separation of the parenchyma. The tensions may be associated with procedures (e.g., like laparoscopic or robotic colorectal anastomosis), where improper staple technique may lead to leaks at the staple line. Stapling techniques may reduce the incidence of intraoperative air leaks and postoperative anastomotic leaks.

The system may use sensors to detect device closure and tip stability. Bluetooth communication may transfer data to an external hub or system (e.g., Ottava). External monitoring systems such as local visualization scopes or tissue strain optical measurement systems may notify the user of irregular tissue folds, variations in compression, or non-homogeneity between tissue layers or planes. When the stapler or energy device is clamped and not yet fired, the system may recommend repositioning. The device may suggest adjusting the rate and pauses to handle irregular tissue sections, minimizing complications by controlling the speed of advancement, allowing for more local compression, or extending compression times.

Features described herein may be associated with control of micro tissue tension due to jaw interactions with tissue layers. The system may control micro tissue tension resulting from the interaction between the jaws of devices (e.g., a dual jaw RF Advanced Bipolar device, Endocutter, or circular stapler) and the tissue layers they engage. When the devices oppose tissue and compress tissue prior to applying therapy, the manner in which the tissue is handled in the jaws may impact the treatment outcome. For example, when tissue is compressed in the jaws of an Endocutter, overloading the proximal end of the jaws may lead to layers of tissue folding over themselves. In examples, where the device is intended to compress two layers, the overloading may result in compressing four layers instead.

As the I-beam or progressive pressure deployment mechanism is actuated from the proximal to distal end, the uneven distribution of tissue within the jaws may create variable tissue compression during the staple deployment. This irregular compression may cause non-uniform staple formation, irregular staple height, and uneven residual tissue compression across the staple line. Such inconsistencies in tissue remodeling may lead to bleeding, tissue leakage, or improper healing over the staple line, such as in sensitive areas like the gastric or colon tissues.

Circular stapler application may be associated with tissue layer irregularity. In examples associated with a circular stapler, the device may fire through one or more temporary staple lines. The circular anastomosis may reconnect tubular structures, such as the colon, with portions of the circular line extending over previously deployed staples. The circular line may overlap four layers of tissue, and other portions involve two layers. The result may be a non-uniform tissue thickness, leading to discrepancies in staple height and compression.

Dog-ear portions, where four layers of tissue and pre-existing staples are present, may lead to irregular compression and structural instability. This may accelerate localized tissue death, which may occur before sufficient tissue remodeling has taken place. In a colon procedure, for example, the circular staple line may be designed to slough off and pass through the body within 12-18 days. If tissue death occurs prematurely, before the colon has adequately remodeled, the outcome may be a colorectal post-surgical leak into the abdomen, potentially leading to sepsis.

Features described herein may be associated with limiting or scaling of device control motions to improve end-effector tip stability. The system may limit or scale device control motions to affect (e.g., improve) the stability of the end-effector tip, preventing unintended coupled motions, such as tremors. By controlling the device's precision during the procedure, the system may track the movement of the circular device components, allowing the user to maintain stable and controlled actions. The tracking of movement may reduce errors caused by shaky or imprecise movements, which may make it easier for the user to guide the device and perform tasks with greater accuracy.

Features described herein may be associated with utilizing movement tracking for circular device components. To assist in device control, the system may utilize tracking mechanisms to follow the movement of circular device components. The movement tracking may be associated with simplification of guidance in the procedure.

Tissue planes beyond remodeling may be detected. When the system encounters areas where tissue remodeling obscures the original tissue planes, the system may project previously monitored tissue planes that have been adapted based on the general anatomical layout for the population. The projections may highlight probable locations of critical structures to guide the surgeon. The system may utilize local ultrasound imaging to identify connective tissues interconnecting the organ tissues on a side of a tissue plane. The ultrasound imaging may allow for the identification of tissue planes that are otherwise not visible to the naked eye.

Tissue scarring may be identified. The system may offer functionality for detecting and identifying scarred tissue, both during the preoperative planning phase and intraoperatively. In preoperative planning, prior surgeries may indicate the presence of scarring in the targeted area based on external imaging. During the surgical procedure, the surgeon may visually identify scarring, and/or the system's instruments may detect electrical properties that differentiate scarred tissue from healthy tissue. Furthermore, advanced visualization techniques incorporated into the system may provide distinctions between scarred and non-scarred tissue.

A fissure between the lobes in a lobectomy may be dissected. In a lobectomy, the system may assist with dissecting the fissure between lung lobes, especially in patients with a history of surgery, pneumonia, interstitial lung disease, or tuberculosis. Chronic tuberculosis may cause continuous tissue remodeling, resulting in layers of scar tissue that obscure natural tissue planes. The planes may be followed to avoid damaging critical structures, and scar tissue may affect an ability to locate the critical structures. Tuberculosis may increase the likelihood of cancer or require surgical intervention, which may add complexity to the procedure.

The surgeon may navigate down to arteries and veins, which may be transected before dividing the parenchyma. When tissue planes are unclear due to scarring, the system may use multi-spectral imaging to identify remnants of the planes. The multi-spectral imaging may allow the surgeon to advance more aggressively along the dissection path without causing collateral damage. By reconstructing the planes, the system may help avoid inadvertent injury to vital structures, improving precision and outcomes in complex cases.

The tissue plane may be indicated, and it may be determined how to re-access the tissue plane. The system may provide an indication of the tissue plane and methods for re-accessing the tissue plane when the plane becomes convoluted or obscured. The system may identify the smallest or most aligned convoluted tissue path for dissection to re-establish access to the normal anatomical tissue plane. Determining that there are no underlying vessels or critical structures in the convoluted tissue plan portion may be associated with avoiding injury. The indicated dissection path may be adjusted based on detected underlying critical structures or suspected structures. The adjustments may be made by overlaying an adjusted pre-operative full-body CT scan with locally identified landmarks and registration points, providing the surgeon with a detailed map of where critical structures are likely to reside.

When portions of the tissue plane are obscured, the system may rely on portions of the tissue plane that have already been navigated or dissected, as well as portions of the plane that remain deeper within the tissue. The system may virtually define where the tissue plane should be, allowing the surgeon to follow this projected route until the tissue plane visually re-emerges. Such real-time guidance may help the surgeon navigate convoluted tissue paths without risking damage to critical structures or underlying vessels.

Instrument control forces may be adapted based on an identified task type or surgical job. The system may adapt instrument control forces based on the type of tissue and the surgical task being performed. For example, where tissue does not have a natural separation plane, such as the liver, the system may mechanically create a separation path. The energy device may semi-cauterize the tissue during dissection, relying on force-driven techniques to complete the procedure.

Features described herein may be associated with atraumatic tissue separation. The system may enable atraumatic tissue separation by limiting the insertion load of instruments (e.g., a dual jaw dissector). The amount of force applied during insertion may be carefully controlled to determine that the tissue is not damaged. The system may monitor tissue displacement relative to the force applied, allowing the surgeon to assess whether a significant amount of force has been exerted for a known displacement.

Anatomically contextualized force limitations may be based on use and anatomy. The system may adapt tissue interaction forces based on both the type of tissue being operated on and the specific surgical job. For example, during dissection or retraction, the force applied by the instrument may be adjusted to suit the tissue type. In examples, delicate tissues may require lower force thresholds to avoid tearing or trauma. Tougher or more fibrous tissues may require higher forces to achieve the desired separation. The system may consider anatomical context, using force limitations that are tailored to the specific tissue and surgical task.

A robotic procedure force adjustment may be based on tissue type. In a robotic procedure, a robotic arm may be used to push a solid organ out of the way to allow visibility for a dissection to occur. Since the solid organ does not pose an immediate risk of damage, a larger force may be applied. When utilizing a grasper for cancerous or delicate tissues, such as cancerous tissue, the force applied may be limited to a lower level to minimize the risk of further tissue damage. The system may automatically adjust the force limits depending on the type of tissue.

Force limits may be adapted based on tissue types. The system may adapt force limits based on the specific tissue types involved in the procedure. For example, mesentery tissue, which is thin and able to tear with interspersed vascularity, may require low force levels to prevent tearing or damage. In examples of a solid organ, such as during liver dissection or bifurcation of solid organs, a greater amount of force may be permissible. For tissues such as the small bowel or colon, or in colorectal procedures, the system may apply different force limits.

For tissues (e.g., like the esophagus), which include repeating cartilaginous rings providing radial expansive force and interconnecting connective tissues, compressive and puncture properties may be present. In examples, the system may adjust force limits using multi-spectral or visible spectrum visualization systems or through electrical impedance readings. The inputs (e.g., multi-spectral or visible spectrum visualization systems/electrical impedance readings) may allow the system to differentiate tissue types and adjust force parameters accordingly.

Powered actuations and energy applications may be adapted based on tissue configuration. The system may adjust powered actuations and advanced energy applications based on the tissue type and configuration detected during the procedure. The system may monitor the device's performance in separating the tissue and adjust accordingly to maintain safety. For example, softer or vascular tissues may require (e.g., lower) force thresholds, and tougher tissues may allow for (e.g., more) force or energy application.

In examples, the surgical system may determine a movement direction and a movement speed associated with the surgical cutting instrument as the surgical cutting instrument cuts the second target tissue. The system may determine, based on the identified current task, a coupled displacement associated with the surgical cutting instrument and the retraction instrument. The system may determine, based on the movement direction associated with the surgical cutting instrument, the movement speed associated with the surgical cutting instrument and the coupled displacement, a motion associated with the retraction instrument. The system may generate a control signal configured to instruct the retraction instrument to move in accordance with the determined motion.

In examples, the surgical system may determine a movement direction associated with the surgical cutting instrument as the surgical cutting instrument cuts the second target tissue. The system may generate a control signal configured to indicate the determined movement direction associated with the surgical cutting instrument and the calculated retraction force direction associated with the retraction instrument on a surgical site display.

In examples, the surgical system may determine a current movement direction and a current movement speed associated with the surgical cutting instrument as the surgical cutting instrument cuts the second target tissue. The system may predict, based on the current movement direction and the current movement speed associated with the surgical cutting instrument, a cutting location of the surgical cutting instrument at a future time instance. The system may calculate, based on the cutting location of the surgical cutting instrument at a future time instance, a target motion of the retraction instrument and a target retraction force at the future time instance. The system may generate a control signal configured to indicate the determined target motion of the retraction instrument and the target retraction force at the future time instance on a surgical site display.

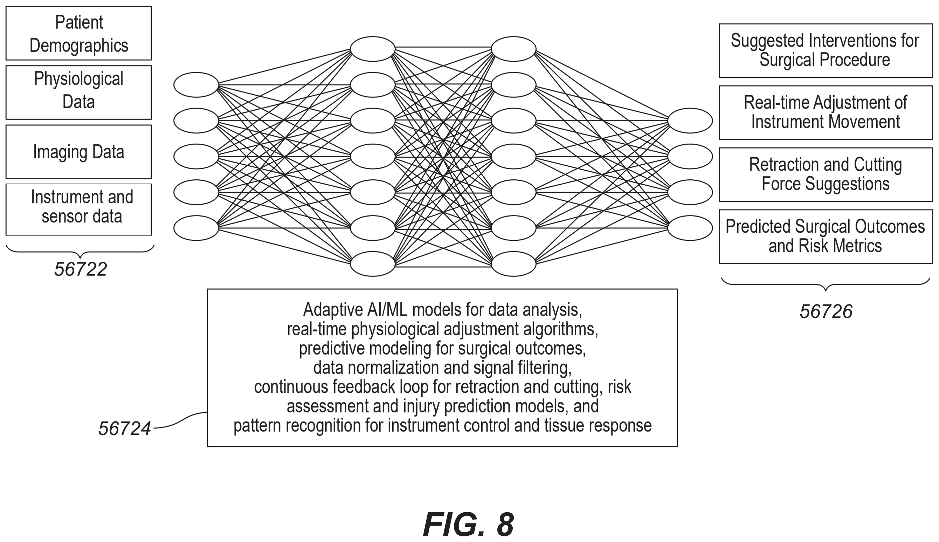

FIG. 8 illustrates a machine learning-based system for managing surgical procedures by processing input data through stages of input, AI/ML processing, and output to facilitate real-time adjustments and decision-making during surgeries. This system may affect the precision and safety of tissue retraction and cutting by analyzing input data, applying machine learning models, and producing interventions for the surgical environment. The system may adjust instrument movement and force based on real-time physiological feedback and predictive modeling.

At 56722, the system may gather input data from various sources, including user goals, patient demographics, physiological data determined prior to the surgical procedure, imaging data, instrument and sensor input data from surgical instruments, and real-time user engagement information. The data may include information about the surgical environment, such as tissue type, instrument position, and user preferences. The sensor data may monitor physiological signals or the status of the surgical instruments.

At 56724, the system may use machine learning models to process the input data. The system may perform real-time physiological adjustment, predictive modeling for surgical outcomes, and use feedback loops for the retraction and cutting of tissues. Machine learning algorithms may be applied to detect patterns and assess risk to predict possible tissue injury and recommend adjustments in the surgical instruments' direction, speed, and force.

At 56726, the system may produce output data that directs interventions during the surgical procedure. Based on the insights generated at 56724, the system may suggest real-time adjustments to instrument movement, cutting force, and retraction force. The system may provide predictive metrics on potential risks and likely outcomes. The systems and methods described herein may be capable of generating a plurality of control signals to serve more than one specific purpose, and references to generating control signals for fulfilling one or more functions need not be read as mutually exclusive. For instance, a second claim referencing "generating a control signal" that is dependent on a first claim, also referencing "generating a control signal", may be construed as if said second claim specified generating a first control signal and a second control signal (corresponding to that of the first and second claim respectively) unless prohibited by context. It will be appreciated that all present dependent claims may readily be so construed when read in combination with one another, where context permits.

The following list of numbered Examples forms part of the present disclosure:
1. A surgical system comprising:
   a processor configured to:
   identify a current task associated with a surgical cutting instrument configured to cooperate with a retraction instrument;
   determine a proximity of a neighboring structure relative to the retraction instrument;
   calculate, based on the identified current task and the determined proximity of the neighboring structure, a retraction force direction and a retraction force intensity associated with the retraction instrument for retracting a first target tissue to facilitate cutting of a second target tissue by the surgical cutting instrument; and
   generate a control signal configured to instruct the retraction instrument to exert a retraction force in the calculated retraction force direction with the calculated retraction force intensity.
2. The surgical system of Example 1, wherein the processor is further configured to:
   determine a movement direction and a movement speed associated with the surgical cutting instrument as the surgical cutting instrument cuts the second target tissue at a first time instance;
   calculate a target displacement of the first target tissue at a second time instance based on the movement direction and the movement speed associated with the surgical cutting instrument;
   calculate, based on the target displacement of the first target tissue and the proximity of the neighboring structure relative to the retraction instrument, a probability that retracting the first target tissue to the target displacement would injure the neighboring structure; and
   generate a second control signal to modify the retraction force based on the calculated probability.
3. The surgical system of Example 2, wherein the processor is further configured to:
   generate a third control signal to instruct the surgical cutting instrument to modify at least one of the movement direction or the movement speed.
4. The surgical system of Example 2, wherein the processor is further configured to:
   generate an indication configured to indicate a likelihood of injury to the neighboring structure caused by the retraction instrument should the surgical cutting instrument maintain the current movement direction and the current movement speed.
5. The surgical system of Example 1, wherein the processor is further configured to:
   determine, based on the identified current task associated with the surgical cutting instrument, a target tissue tension range associated with the second target tissue; and
   determine a maximum retraction force intensity based on the determined target tension range associated with the second tissue to avoid tearing of the second target tissue, wherein the retraction force intensity associated with the retraction instrument is calculated based on the determined maximum retraction force intensity.
6. The surgical system of Example 1, wherein the processor is further configured to:
   determine, based on the identified current task associated with the surgical cutting instrument, a target displacement associated with the first target tissue such that the second target tissue is visible for cutting by the surgical cutting instrument; and
   determine a minimum retraction force intensity based on the determined target displacement associated with the first target tissue, wherein the retraction force intensity associated with the retraction instrument is calculated based on the determined minimum retraction force intensity.
7. The surgical system of Example 1, wherein the processor is further configured to:
   obtain a preselected force intensity limit associated with the retraction instrument, wherein the retraction force intensity associated with the retraction instrument is calculated based further based on the preselected force intensity limit.
8. The surgical system of Example 1, wherein the processor is further configured to:
   determine a movement direction and a movement speed associated with the surgical cutting instrument as the surgical cutting instrument cuts the second target tissue;
   determine, based on the identified current task, a coupled displacement associated with the surgical cutting instrument and the retraction instrument;
   determine, based on the movement direction associated with the surgical cutting instrument, the movement speed associated with the surgical cutting instrument and the coupled displacement, a motion associated with the retraction instrument; and
   generate a control signal configured to instruct the retraction instrument to move in accordance with the determined motion.
9. The surgical system of Example 1, wherein the processor is further configured to:
   determine a movement direction associated with the surgical cutting instrument as the surgical cutting instrument cuts the second target tissue; and
   generate a control signal configured to indicate the determined movement direction associated with the surgical cutting instrument and the calculated retraction force direction associated with the retraction instrument on a surgical site display.
10. The surgical system of Example 1, wherein the processor is further configured to:
   determine a current movement direction and a current movement speed associated with the surgical cutting instrument as the surgical cutting instrument cuts the second target tissue;
   predict, based on the current movement direction and the current movement speed associated with the surgical cutting instrument, a cutting location of the surgical cutting instrument at a future time instance;
   calculate, based on the cutting location of the surgical cutting instrument at a future time instance, a target motion of the retraction instrument and a target retraction force at the future time instance; and
   generate a control signal configured to indicate the determined target motion of the retraction instrument and the target retraction force at the future time instance on a surgical site display.
11. A method for a surgical system, the method comprising:
   identifying a current task associated with a surgical cutting instrument configured to cooperate with a retraction instrument;
   determining a proximity of a neighboring structure relative to the retraction instrument;
   calculating, based on the identified current task and the determined proximity of the neighboring structure, a retraction force direction and a retraction force intensity associated with the retraction instrument for retracting a first target tissue to facilitate cutting of a second target tissue by the surgical cutting instrument; and
   generating a control signal configured to instruct the retraction instrument to exert a retraction force in the calculated retraction force direction with the calculated retraction force intensity.
12. The method of Example 11, wherein the method further comprises:
   determining a movement direction and a movement speed associated with the surgical cutting instrument as the surgical cutting instrument cuts the second target tissue at a first time instance;
   calculating a target displacement of the first target tissue at a second time instance based on the movement direction and the movement speed associated with the surgical cutting instrument;
   calculating, based on the target displacement of the first target tissue and the proximity of the neighboring structure relative to the retraction instrument, a probability that retracting the first target tissue to the target displacement would injure the neighboring structure; and
   generating a second control signal to modify the retraction force based on the calculated probability.
13. The method of Example 12, wherein the method further comprises:
   generating a third control signal to instruct the surgical cutting instrument to modify at least one of the movement direction or the movement speed.
14. The method of Example 12, wherein the method further comprises:
   generating an indication configured to indicate a likelihood of injury to the neighboring structure caused by the retraction instrument should the surgical cutting instrument maintain the current movement direction and the current movement speed.
15. The method of Example 11, wherein the method further comprises:
   determining, based on the identified current task associated with the surgical cutting instrument, a target tissue tension range associated with the second target tissue; and
   determining a maximum retraction force intensity based on the determined target tension range associated with the second tissue to avoid tearing of the second target tissue, wherein the retraction force intensity associated with the retraction instrument is calculated based on the determined maximum retraction force intensity.
16. The method of Example 11, wherein the method further comprises:
   determining, based on the identified current task associated with the surgical cutting instrument, a target displacement associated with the first target tissue such that the second target tissue is visible for cutting by the surgical cutting instrument; and
   determining a minimum retraction force intensity based on the determined target displacement associated with the first target tissue, wherein the retraction force intensity associated with the retraction instrument is calculated based on the determined minimum retraction force intensity.
17. The method of Example 11, wherein the method further comprises:
   obtaining a preselected force intensity limit associated with the retraction instrument, wherein the retraction force intensity associated with the retraction instrument is calculated based further based on the preselected force intensity limit.
18. The method of Example 11, wherein the method further comprises:
   determining a movement direction and a movement speed associated with the surgical cutting instrument as the surgical cutting instrument cuts the second target tissue;
   determining, based on the identified current task, a coupled displacement associated with the surgical cutting instrument and the retraction instrument;
   determining, based on the movement direction associated with the surgical cutting instrument, the movement speed associated with the surgical cutting instrument and the coupled displacement, a motion associated with the retraction instrument; and
   generating a control signal configured to instruct the retraction instrument to move in accordance with the determined motion.
19. The method of Example 11, wherein the method further comprises:
   determining a movement direction associated with the surgical cutting instrument as the surgical cutting instrument cuts the second target tissue; and
   generating a control signal configured to indicate the determined movement direction associated with the surgical cutting instrument and the calculated retraction force direction associated with the retraction instrument on a surgical site display.
20. The method of Example 11, wherein the method further comprises:
   determining a current movement direction and a current movement speed associated with the surgical cutting instrument as the surgical cutting instrument cuts the second target tissue;
   predicting, based on the current movement direction and the current movement speed associated with the surgical cutting instrument, a cutting location of the surgical cutting instrument at a future time instance;
   calculating, based on the cutting location of the surgical cutting instrument at a future time instance, a target motion of the retraction instrument and a target retraction force at the future time instance; and
   generating a control signal configured to indicate the determined target motion of the retraction instrument and the target retraction force at the future time instance on a surgical site display.

## Claims

1. A surgical system comprising:
a processor configured to:
identify a current task associated with a surgical cutting instrument configured to cooperate with a retraction instrument;
determine a proximity of a neighboring structure relative to the retraction instrument;
calculate, based on the identified current task and the determined proximity of the neighboring structure, a retraction force direction and a retraction force intensity associated with the retraction instrument for retracting a first target tissue to facilitate cutting of a second target tissue by the surgical cutting instrument; and
generate a control signal configured to instruct the retraction instrument to exert a retraction force in the calculated retraction force direction with the calculated retraction force intensity.

2. A computer-readable medium comprising instructions which, when executed by a computer, cause the computer to:
identify a current task associated with a surgical cutting instrument configured to cooperate with a retraction instrument;
determine a proximity of a neighboring structure relative to the retraction instrument;
calculate, based on the identified current task and the determined proximity of the neighboring structure, a retraction force direction and a retraction force intensity associated with the retraction instrument for retracting a first target tissue to facilitate cutting of a second target tissue by the surgical cutting instrument; and
generate a control signal configured to instruct the retraction instrument to exert a retraction force in the calculated retraction force direction with the calculated retraction force intensity.

3. The surgical system of claim 1 or computer-readable medium of claim 2, wherein the processor is further configured to:
determine a movement direction and a movement speed associated with the surgical cutting instrument as the surgical cutting instrument cuts the second target tissue at a first time instance;
calculate a target displacement of the first target tissue at a second time instance based on the movement direction and the movement speed associated with the surgical cutting instrument;
calculate, based on the target displacement of the first target tissue and the proximity of the neighboring structure relative to the retraction instrument, a probability that retracting the first target tissue to the target displacement would injure the neighboring structure; and
generate a second control signal to modify the retraction force based on the calculated probability.

4. The surgical system or computer-readable medium of claim 3, wherein the processor is further configured to, or the instructions further cause the computer to:
generate a third control signal to instruct the surgical cutting instrument to modify at least one of the movement direction or the movement speed.

5. The surgical system or computer-readable medium of claim 3 or 4, wherein the processor is further configured to, or the instructions further cause the computer to:
generate an indication configured to indicate a likelihood of injury to the neighboring structure caused by the retraction instrument should the surgical cutting instrument maintain the current movement direction and the current movement speed.

6. The surgical system or computer-readable medium of any preceding claim, wherein the processor is further configured to, or the instructions further cause the computer to:
determine, based on the identified current task associated with the surgical cutting instrument, a target tissue tension range associated with the second target tissue; and
determine a maximum retraction force intensity based on the determined target tension range associated with the second tissue to avoid tearing of the second target tissue, wherein the retraction force intensity associated with the retraction instrument is calculated based on the determined maximum retraction force intensity.

7. The surgical system or computer-readable medium of any preceding claim, wherein the processor is further configured to, or the instructions further cause the computer to:
determine, based on the identified current task associated with the surgical cutting instrument, a target displacement associated with the first target tissue such that the second target tissue is visible for cutting by the surgical cutting instrument; and
determine a minimum retraction force intensity based on the determined target displacement associated with the first target tissue, wherein the retraction force intensity associated with the retraction instrument is calculated based on the determined minimum retraction force intensity.

8. The surgical system or computer-readable medium of any preceding claim, wherein the processor is further configured to, or the instructions further cause the computer to:
obtain a preselected force intensity limit associated with the retraction instrument, wherein the retraction force intensity associated with the retraction instrument is calculated based further based on the preselected force intensity limit.

9. The surgical system or computer-readable medium of any preceding claim, wherein the processor is further configured to, or the instructions further cause the computer to:
determine a movement direction and a movement speed associated with the surgical cutting instrument as the surgical cutting instrument cuts the second target tissue;
determine, based on the identified current task, a coupled displacement associated with the surgical cutting instrument and the retraction instrument;
determine, based on the movement direction associated with the surgical cutting instrument, the movement speed associated with the surgical cutting instrument and the coupled displacement, a motion associated with the retraction instrument; and
generate a control signal configured to instruct the retraction instrument to move in accordance with the determined motion.

10. The surgical system or computer-readable medium of any preceding claim, wherein the processor is further configured to, or the instructions further cause the computer to:
determine a movement direction associated with the surgical cutting instrument as the surgical cutting instrument cuts the second target tissue; and
generate a control signal configured to indicate the determined movement direction associated with the surgical cutting instrument and the calculated retraction force direction associated with the retraction instrument on a surgical site display.

11. The surgical system or computer-readable medium of any preceding claim, wherein the processor is further configured to, or the instructions further cause the computer to:
determine a current movement direction and a current movement speed associated with the surgical cutting instrument as the surgical cutting instrument cuts the second target tissue;
predict, based on the current movement direction and the current movement speed associated with the surgical cutting instrument, a cutting location of the surgical cutting instrument at a future time instance;
calculate, based on the cutting location of the surgical cutting instrument at a future time instance, a target motion of the retraction instrument and a target retraction force at the future time instance; and
generate a control signal configured to indicate the determined target motion of the retraction instrument and the target retraction force at the future time instance on a surgical site display.
